Europäisches Patentamt

European Patent Office

Office européen des brevets

(11) Publication number: **0 073 192**
**B1**

# (12) EUROPEAN PATENT SPECIFICATION

(45) Date of publication of patent specification: **18.01.89**

(51) Int. Cl.⁴: **A 61 F 5/04**

(21) Application number: **81901934.0**

(22) Date of filing: **09.02.81**

(86) International application number:
**PCT/US81/00166**

(87) International publication number:
**WO 82/02658 19.08.82 Gazette 82/20**

(54) **IMPROVED MODIFIED ORTHOTIC DEVICE.**

(43) Date of publication of application:
**09.03.83 Bulletin 83/10**

(45) Publication of the grant of the patent:
**18.01.89 Bulletin 89/03**

(84) Designated Contracting States:
**CH DE FR GB LI NL SE**

(56) References cited:
**DE-A-2 724 586**
**FR-A-2 454 295**
**US-A- 58 403**
**US-A- 901 592**
**US-A-3 885 252**
**US-A-3 902 482**

(73) Proprietor: **ANDERSON, George Clarence**
**2883 Doidge Avenue**
**Pinole, CA 94564 (US)**

(72) Inventor: **ANDERSON, George Clarence**
**2883 Doidge Avenue**
**Pinole, CA 94564 (US)**

(74) Representative: **Smith, Sydney et al**
**Elkington and Fife High Holborn House 52/54**
**High Holborn**
**London WC1V 6SH (GB)**

Courier Press, Leamington Spa, England.

EP 0 073 192 B1

**Description**

## BACKGROUND OF THE INVENTION

This invention relates to an improved orthotic device for use in a new assembly of knee stabilizer and modified cast brace. More particularly, this invention relates to a new and improved double hinged single side brace for use in support and alignment of the joints, security of limb position, prevention of change in position attributed to rotatory forces on the fracture site, prevention of swelling complications, and economy of physician's time. Support and protection is to the medial collateral ligament of lateral collateral ligament of the knee.

The knee-joint is made up of two condyloid joints and a third joint, partly arthrodial, but not completely so, since the articular surfaces are not mutually adapted to each other, so that the movement is not a simple gliding one. The principal movements that take place at the knee-joint are flexion and extension. The movements of flexion and extension at this joint differ from those in a typical hinge joint, such as the elbow, in that the axis around which motion takes place is not a fixed one but shifts forward during extension, as a gliding movement is superposed on the rolling, and shifts backward during flexion.

Although the knee-joint has been described as a hinge joint, it is really of a much more complicated character. It must be regarded as consisting of three articulations, of two different kinds. The first kind is a condyloid articulation; in this form of joint, an ovoid articular surface, or condyle, is received into an elliptical cavity in such a manner as to permit flexion, extension, abduction, adduction, and circumduction, but no axial rotation. The second kind of articulation involved is arthrodial; this is a joint which permits only gliding movement. It is formed by the apposition of plane surfaces, or one slightly concave, the other slightly convex, the amount of motion between them being limited by the ligaments or osseous processes surrounding the articulation.

Persons who have sustained knee injuries, who have had operations to remove cartilage, or who have weak knee-joints from causes, such as arthritis or athletes who have sustained an injury to the medial collateral ligament or lateral collateral ligament of the knee, need protection principally against lateral motion of the knee, that is, motion may be the result, for example, of a blow to the side of the knee. At the same time, a suitable knee brace should not interfere with the normal flexion and extension of the leg. The brace should protect the knee against sidewise motions during both flexion and extension, this means that the bracing structure should continue to lie parallel to the parts of the leg above and below the knee-joint in all positions of the brace structure and should remain substantially at the knee and provide protection to the knee.

The knee-joint has four principal ligaments, one on either side and two on the inside. There ligaments may be strained or torn in sports and accidents. Injuries to these ligaments can be serious and must be properly treated if disability is to be avoided. Above all, repeated injury or strains before healing must be avoided. All degrees of ligament injuries will lead to some atrophy of the quadriceps and hamstring muscle groups.

Many previous orthotic devices for use as knee braces and protection devices have been simple hinged structures pivotable about a fixed point, which cannot move parallel to the complex motion of the knee joint. Some knee braces for support and protection of the knee-joint comprises both an inner and outer bracing structure each a rigid planar and elongated arm and pivoted about a fixed point. The fixed point lies on and parallel to the knee and leg. During motion and a sideways blow, the rigid planar arms may cause further injury or discomfort to the already injured ligaments of the knee-joint.

Some of the previous knee braces fail to provide protection to the injured knee ligaments. While other braces may lie parallel to the leg and knee-joint while it is extended when the leg is flexed, the bracing structure fails to follow the motion accurately.

The instant improved orthotic device may be used in cast bracing for derotational or knee bracing; also used to distribute the load of functional below-the-knee fracture bracing. Cast bracing stresses proper alignment of the joints, security of limb position, prevention of swelling complications and economy of physician's time. A special feature is a double hinged single side brace utilized in paired configuration on each side of the knee-joint. Within the features of the improved modified cast brace of this invention is a modified double hinged double axis joint that restricts the arc of the leg motion to safe limits, generally 20 to 90 degrees. Functionally, the use of the modified cast brace joint compares favorably with previous treatments. Early motion was extremely well tolerated, the stability of operated knees was well supported and aided in management of serious knee ligament injuries.

Clinical and laboratory evidence has proven that prolonged immobilization is detrimental to synovial joints. Traditional treatment of knee-joint and leg injuries commonly involves immobilization for six to eight weeks. Periods of immobilization of this length produce alterations in the biochemistry of connective tissue, the micro- and ultra-structure of synovial membrane, cartilage, tendon, and in the biomechanical properties of the ligament-bone unit. Muscle atrophy and vascular thrombi are common extra-articular complications of prolonged immobilization.

Means for minimizing these undesirable effects are desirable. Therefore, early protected motion will result in minimizing the undesirable effects. The instant invention is a specific modified cast brace which allows limited knee motion yet protects healing structures from disruptive stresses.

## SUMMARY OF THE INVENTION

Objects of the present invention are to provide for an improved modified orthotic-cast brace assembly which permits and provides freedom of movement of the knee during flexion and extension of the leg in a cast without restriction or physical discomfort during movement.

Another object of the present invention is to provide an improved modified orthotic-cast brace assembly which permits and provides freedom of movement of the knee during flexion and extension of the leg in a cast without restriction or physical discomfort during movement.

Another object of the present invention is to provide an improved modified orthotic-cast brace assembly which permits and provides for use on an already injured knee and provides prevention of further injury or strain.

Another object of the present invention is to provide a paired configuration of rigid double hinged single side orthotic-knee brace pivotally hinged to upper and lower flanges and attachment wings for easy securing and removal to the cast material on the wearer's leg.

It is another object to provide such a modified orthotic-cast brace and stabilizer that does not interfere with normal extension and flexion of the wearer's leg while in the cast, while preventing sidewise motion of the knee-joint whether the leg is flexed or extended giving added support and comfort thereto and providing rotational stability.

From DE-A-2,724,586, there is known an orthotic device comprising a rigid center support member having an upper end portion and a lower end portion, an upper support member non-slidably pivotally hinged to the upper end portion of the center support member and a lower support member non-slidably pivotally hinged to the lower end portion of the center support member. The upper and lower ends of the center support member are adjacent the upper and lower support members at the respective hinge locations. As disclosed in FR-A-2,454,295, for example, such devices may be applied by means of casts rather than straps.

According to the present invention, the problem of avoiding contact between the center support member and the knee is obviated by an off-set arrangement of the center support member.

In one embodiment, the present improvement is characterised in that the said rigid center support member also has a central portion, the general plane of the central portion being laterally offset from those of the end portions.

In another embodiment, the present improvement is characterised in that the end portions of the upper and lower support members are each laterally offset from the respective general planes of the upper and lower support members.

In either embodiment, the upper and lower support members may be embedded in casting material to form a cast brace. Attachment wings each separately and individually securely attached to the upper and lower support members may be provided. Such may be perpendicular to the attached upper and lower support members. Alternatively, the upper and lower support members may have flared configurations to simulate attachment wings. preferably, the rigid center support member is a support bar or rod. The pivotally hinged upper and lower support members may have preset and resettable stop means for limiting knee and leg motion.

The cast brace assembly of one embodiment of the present invention includes a bracing structure comprising a rigid support bar substantially having a concave off-set through its center portion, and at one end pivotally hinged to an upper support member and at the other end pivotally hinged to a lower support member. The center portion having the concave off-set of the rigid support is placed alongside the knee joint.

In preferred embodiments, the present invention encompasses at least one improved orthotic device as a knee stabilizer and derotational for support and protection of a knee-joint and leg, said device described as a double hinged single side brace comprising a rigid support structure having an off-set design through its center portion. Butt-joint hinges provide a less cumbersome hinge arrangement. Said offset is optionally formed by bending of the center support member, or by bending the upper and lower support members and connecting therebetween on the raised portion of the straight center support member. The upper support member to which the upper end of the rigid center support member is pivotally hinged may be embedded in the cast material above the knee-joint for movement with the upper leg and limited motion parallel to the upper leg, and lower support member to which the lower end of the rigid center support member pivotally hinged to the wearer's lower leg embedded in the cast material below the knee-joint for movement with the lower leg and limited motion parallel to the lower leg. The support and stabilizing structure has a single rigid center support member having an off-set through its center portion and doubly hinged each at the upper and lower end of the rigid center support member. If the off-set is accomplished by bending the upper and lower support member, then the rigid support bar or rod is flat and straight between the support members. The movement of the leg by normal flexion and extension of the wearer's knee is freely permitted by the double hinged arrangement. The movement comprises any manner consistent with the double hinged action permitted by the rigid center support member and normal to the plane of the rigid center support member, thereby preventing sidewise motion of the knee-joint.

Other objects, features and advantages will appear from the following description of a preferred embodiment of the invention, taken together with the attached drawings, in which:

The improved orthotic device assembly consists essentially of a bracing structure consisting essentially of a rigid support bar or rod substantially having a center portion; at each end of said center portion, there is pivotally hinged thereto extension support member. Securely attached to each extension support member there are optionally attachment wings. The attachment wings are used to assist in positioning and embedding the extension support members into the casting material to secure the cast brace to the casting material. Alternative to the optional attachment wings, the extension support member itself may be flared to simulate the attachment wings. The support members can be contoured with bending irons to allow flush apposition to the cast.

FIG. 1 generally shows an orthotic device adapted in a cast brace in paired configuration in place with respect to the extended leg of the wearer;

FIG. 2 generally shows an orthotic device in a modified cast brace in relation to the flexed leg of the wearer;

FIG. 3 is a general perspective view of an orthotic device;

FIG. 4 is a detailed view of a stop means for regulating or restricting movement in an orthotic device;

FIG. 5 is a perspective view of alternative arrangements for positioning the center support bar or rod, c and d being in accordance with the present invention; and

FIG. 6 is a general perspective view of alternative flared flanges for attachment.

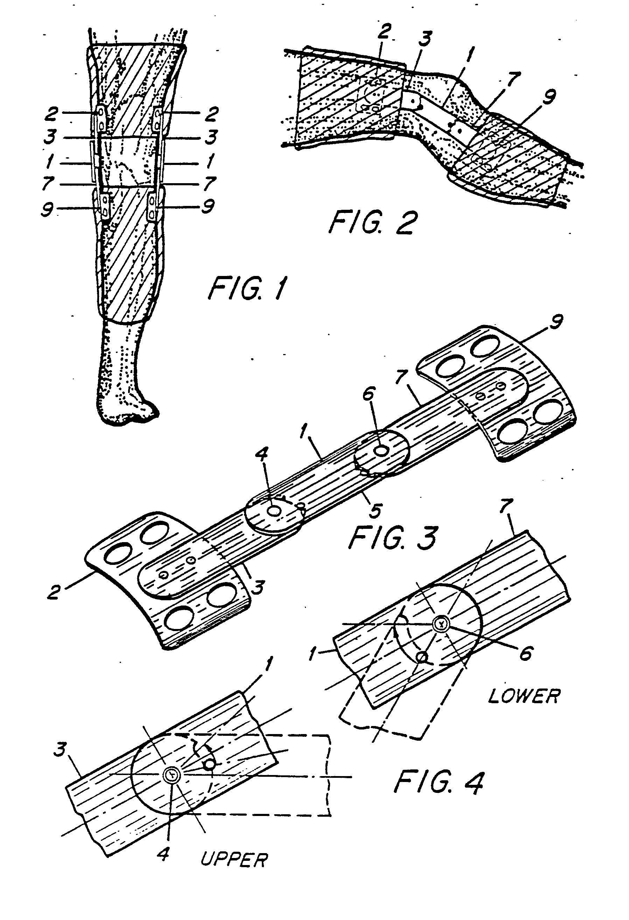

Referring to the drawings, FIGS. 1-6, an orthotic device includes a single rigid support bar or rod 1 having through the center portion a support bar or rod 5. The support bar or rod 5 has two end portions which are continuations of the center support bar or rod. Each end portion is provided with an opening which accommodates a means for pivotally fastening 4 and 6 to each end portion of the support bar or rod to extension support members 3 and 7. The support members 3 and 7 are pivotally fastened at 4 and 6 to each end portion of the rigid support bar or rod 5. Such pivotal connection is essential to the function of the cast brace, as discussed in the following.

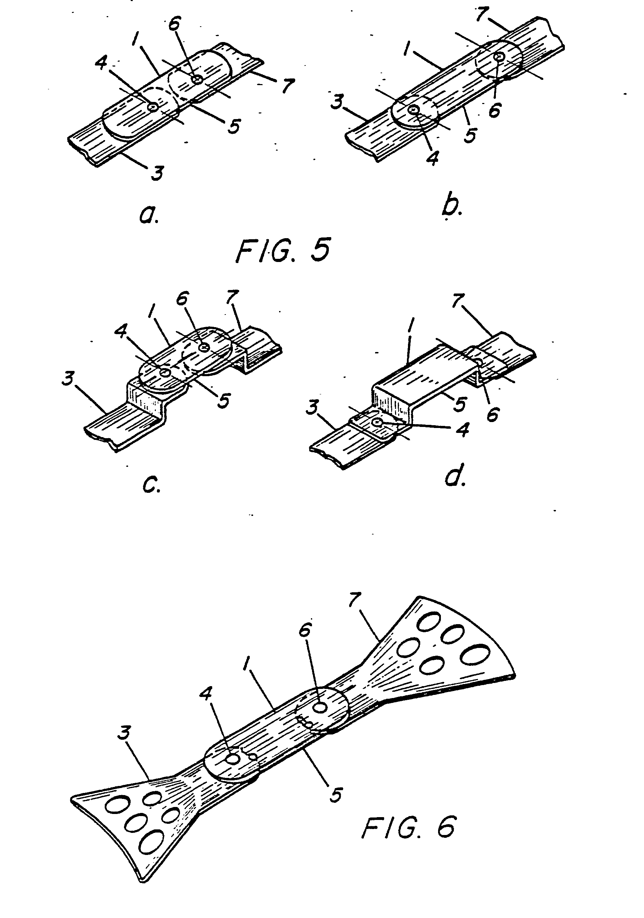

Each of the support members 3 and 7 are terminated integrally with optional attachment wings 2 and 9. Each attachment wing is provided perpendicular to the connecting support member. Alternatively, the support members may be flared to simulate the attachment wings as in FIG. 6. Referring to FIG. 1 and FIG. 2, an orthotic device is in place on the wearer's leg. The support members 3 and 7 terminated by attachment wings 2 and 9 or flaring of the support members are generally parallel to the upper and lower parts of the wearer's leg even at flexion or extension. The support members and optional attachment wings which are securely fastened to each other or using flared support members, are embedded into the casting material. Thereby, the orthotic device is used in a modified cast or fracture brace and becomes an integral part of the cast.

The center portion 5 of the present orthotic device is designed off-set to avoid pressure against the knee-joint of the wearer. As shown in FIG. 5, various alternative configurations are possible for attachment and positioning of the center portion 5, c and d illustrating the present invention. The pivotally mounted support members 3 and 7, wings 2 and 9 or flared support members are securely embedded in the cast material on the wearer's leg, support member and attachment wing, or flared support members, in the upper part of the cast above the knee-joint and support member and attachment wing, or flared support members, in the lower part of the cast below the knee-joint. The three part construction of the double hinged action allows the stabilizer to translate the normal leg action of extension and flexion into the conformed and supported orthotic bracing structure integral with the cast. The combination of motions permitted by the three part construction results in the motion of the attachment wings and support members with respect to the rigid support bar or rod that closely parallels that natural action of the knee-joint. The rigid support bar or rod 1 closely and accurately follows the action of the wearer's knee and at all times continuing to provide support and stability to the knee-joint while the leg is in a cast. Especially protection against sidewise forces and rotational stability is provided.

The improved orthotic device used in a modified cast brace is attachable to the inner or outer leg. Preferably the orthotic device in a modified cast brace is positioned and secured in paired configuration on both sides of the knee opposite the orthotic center of the knee to provide rotational stability. The center portion of the rigid support bar or rod is placed directly over the medial or lateral ligament.

By the double hinge arrangement, the motions of flexion or extension are easily followed by the modified cast brace employing the orthotic device with the accompanying cast support. The operation of the three-segment double hinged arrangement, is best understood by referring to FIG. 1 and FIG. 2. The bracing structure remains generally parallel to the knee-joint, but the action of the joint formed by the stabilizer accurately parallels the action of the individual wearer's knee. At all times, the orthotic device and knee stabilizer of the modified cast brace of this invention remains substantially displaced, but parallel and adjacent the knee-joint, continuing to provide protection and support. Translational motion of the stabilizer of the modified cast brace is limited by being secured into the casting material.

By using the present invention, it is thus possible to have a convenient, effective and comfortable means for stabilizing and supporting the leg and knee-joint and thereby eliminate discomfort or possible injury or reinjury to the leg. More importantly, the improved modified cast brace prevents change in position which otherwise is attributed to rotating forces on the fracture site. Also, the center portion of the rigid support bar or rod of the knee stabilizer of the cast brace is novel in that it does not place pressure on the knee-joint. Acting together, the improved bar or rod and double hinge arrangement with means for restricting movement of the leg form the basis for an original and useful improvement in the design, function and operation of the present orthotic device.

Although a direct comparison between the present construction and other presently known conventional cast brace structures is difficult, it is the opinion of persons who have used the orthotic device in a modified cast brace that more than satisfactory results were obtained. For example, football players, who had each experienced leg injuries, wore the described orthotic device stabilizer and cast brace. By being able to have some motion to the injured leg, rehabilitation and full use of the leg was enhanced. Each player was then able to perform his prescribed duties without extended recuperation. Joint motion and muscle function are maintained so that they are at a functional level at the completion of the treatment.

In use, the orthotic device used in an improved modified cast brace is used in a cast brace assembly which protects healing structures from disruptive stresses on the leg or knee especially rotational forces. In the use, the modified cast brace assembly with the orthotic device is employed in a leg cast on patients with a variety of problems in fractures of the proximal tibia and distal femur. Among the knee and leg problems including acute ligamentous injuries, medial and lateral reconstructions, cruciate ligament prosthesis, total knee arthroplasties, tibial plateau fractures, and patello-femoral arthroplasties.

The improved orthotic device with the stop means for restricting the range of motion of the leg, generally from about 20 to about 90 degrees, the arc and total range of motion may be varied according to the individual pathology. The improved modified cast brace permits isotonic, isometric and progressive resistance exercises while in the cast brace. The cast is worn from six to eight weeks or for a time period as required.

The hinged long leg cast including the double hinged three-segment-brace support is practical from the standpoint of cost, time and ease of application. Also, the comfort to the patient is notable, both in ease of movement when permitted, in weight and functional stability and support. Another distinct advantage of the modified cast brace is the possible reuse of the joint which lessens the cost.

More particularly, a notable advantage to the use of the instant orthotic device in a modified cast brace assembly with the three-segment double hinge brace is the ease of application. Many other cast brace joints and assemblies require the joints need be aligned parallel and over the axis of rotation of the knee. This critical alignment requires an alignment jig — a special tool is required. The instant joint is unique since it does not require special alignment. It is sufficient to place one joint on each side of the knee with the placement reasonably determined over the center of rotation for the knee. Orientation of the joints and extensions contoured to the upper and lower leg curvature allows ease of placement in the cast with maximum comfort to the patient.

When applying the orthotic device in a modified cast brace with the joint in place, plaster may be used as a general cast material. Some of the newer synthetic materials may be more durable with active athletic patients. Also, newer synthetic materials are lighter in weight than plaster. In use of any casting material, the joints are then easily applied to the cast by implanting the extended support members and attachment wings thereto. Means for integral attachment of the orthotic device to the casting material is provided.

Within the embodiment of this invention is the means for regulating and restricting the movement of the cast brace. Because of the ease of movement of the knee fitted with an improved cast brace, including the orthotic device, there is a need for means for regulating and restricting the movement of the leg. Confined movement is often recommended at early stages of healing. Later, freer movement of the knee and leg can be permitted. Therefore, patients with operated cases fail to require longer time for recovery of motion. Repaired knees which "loosen" during immobilization does not apply to cases employing the instant device. Therefore, knees treated with early protected motion show no harm and enhance recovery and early full use of the knee joint.

Also, included within an embodiment of the invention, is the means for angular positioning of the leg. Also, considered is a stop means for limiting the folding movement of the mechanical double hinged joint. There is capability for free movement of the knee, restricted movement and fixed angular positioning of the knee-joint. Varied capability to control the knee and leg movement gives early protected motion, with early limited knee motion, followed by free motion. Actually, motion can be initiated at any time, if the patient has a condition which will permit movement.

The feature of the orthotic device in the modified cast brace is the three-segment double hinged joint. In addition, the modified cast brace may be provided with stop means for adjusting to limit both flexion and extension of the leg and knee-joint, pre-set and resettable means for added freedom in setting a range of knee motions. Setting can range from a locked position to a limited position to a completely adjustable brace to desired limits.

The modified cast brace can be applied with conventional casting materials. For example, if a long leg cast is suitable to the pathology, it can be applied as over a cast sock and elastic knee cage. These items are optional. Additionally, felt pads may be placed over the malleoli, the extensor hallucis longus tendon on the dorsum of the foot, and the peroneal nerve. An additional piece of felt may be placed over the patella to create an identifiable bump for trimming and to turn the edges away from the shin. The modified cast brace with the improved orthotic device in paired configuration is embedded in the casting material on each side of the knee-joint.

The modified cast brace with the improved orthotic device of this invention is useful in many ailments of the leg, knee and knee ligaments. Both pre- and post-operative utility has been realized. Chronic and acute knee and knee ligament injuries can be treated and rehabilitated. In most cases, rehabilitation time after treatment was shortened. Quadriceps and hamstring tone rated fair to good on removal of the modified cast brace. Loosening of repaired knees can be prevented since complete immobilization is not required.

**Claims**

1. An orthotic device for use in knee stabilizers and cast and fracture braces comprising a rigid center support member having an upper end portion and a lower end portion, an upper support member for partially embedding in casting material, non-slidably pivotally hinged to the said upper end portion of the said center support member and a lower support member for partially embedding in casting material, non-slidably pivotally hinged to the said lower end portion of the said center support member, the upper and lower end portions being adjacent the respective support members at the hinge locations, characterised in that the said rigid center support member also has a central portion, the general plane of the central portion being laterally offset from those of the end portions in order to avoid pressure against the knee-joint of a wearer.

2. An orthotic device for use in knee stabilizers and cast and fracture braces comprising a rigid center support member having an upper end portion and a lower end portion, an upper support member for partially embedding in casting material, non-slidably pivotally hinged at a lower end portion thereof to the said upper end portion of the said center support member and a lower support member for partially embedding in casting material, nonslidably pivotally hinged at an upper end portion thereof to the said lower end portion of the said center support member, the general plane of the said rigid center support member being adjacent the end portions of the said upper and lower support members characterised in that the end portions of the upper and lower support members are each laterally offset from the respective general planes of the upper and lower support members in order to avoid pressure against the knee-joint of a wearer.

3. A device as claimed in claim 1 or claim 2 wherein the said upper and lower support members are embedded in casting material.

4. A device as claimed in any of claims 1 to 3 wherein attachment wings each separately and individually securely attached to the said upper and lower support members are provided.

5. A device as claimed in claim 4 wherein the said attachment wings are perpendicular to the said attached upper and lower support members.

6. A device as claimed in any of claims 1 to 5 wherein the said upper and lower support members are flared.

7. A device as claimed in any of claims 1 to 6 wherein the said rigid center support member is a support bar or rod.

8. A device as claimed in any of claims 1 to 7 wherein the said pivotally hinged upper and lower support members have preset and resettable stop means for limiting knee and leg motion.

**Patentansprüche**

1. Orthopädisches Gerät zur Verwendung in Kniestabilisatoren und Gipsverband- und Frakturschienen mit einem starren Zentralstützelement, das einen oberen Endbereich und einen unteren Endbereich aufweist, einem teilweise in Gipsmaterial einzubettenden oberen Stützelement, das unverschiebbar, drehbar an dem oberen Endbereich des Zentralstützelements angelenkt ist, und einem unteren teilweise in Gipsmaterial einzubettenden Stützelement, das unverschiebbar, drehbar an dem unteren Endbereich des Zentralstützelements angelenkt ist, wobei die oberen und unteren Endbereiche an den Gelenkstellen an die entsprechenden Stützelemente angrenzen, dadurch gekennzeichnet, daß das starre Zentralstützelement ferner einen zentralen Bereich aufweist, dessen Hauptebene seitlich gegenüber denen der Endbereiche versetzt ist, um Druck gegen das Kniegelenk des Trägers zu vermeiden.

2. Orthopädisches Gerät zur Verwendung in Kniestabilisatoren und Gipsverband- und Frakturschienen mit einem starren Zentralstützelement, das einen oberen Endbereich und einen unteren Endbereich aufweist, einem oberen teilweise in Gipsmaterial einzubettenden Stützelement, das mit seinem unteren Endbereich unverschiebbar, drehbar an dem oberen Endbereich des Zentralstützelements angelenkt ist, und einem unteren teilweise in Gipsmaterial einzubettenden Stützelement, das mit seinem oberen Endbereich unverschiebbar, drehbar an dem unteren Endbereich des Zentralstützelements angelenkt ist, wobei die ebene Hauptfläche des starren Zentralstützelements an die Endbereiche der oberen und unteren Stützelemente angrenzt, dadurch gekennzeichnet, daß die Endbereiche der oberen und unteren Stützelemente jeweils seitlich gegenüber den entsprechenden Hauptebenen der oberen und unteren Stützelemente versetzt sind, um Druck gegen das Kniegelenk des Trägers zu vermeiden.

3. Gerät nach einem der Ansprüche 1 oder 2, dadurch gekennzeichnet, daß die oberen und unteren Stützelemente in Gipsmaterial eingebettet sind.

4. Gerät nach einem der Ansprüche 1 bis 3, dadurch gekennzeichnet, daß jeweils getrennt und individuell fest mit den oberen und unteren Stützelementen verbundene Befestigungsflügel vorhanden sind.

5. Gerät nach Anspruch 4, dadurch gekennzeichnet, daß die Befestigungsflügel senkrecht zu den damit verbundenen oberen und unteren Stützelementen stehen.

6. Gerät nach einem der Ansprüche 1 bis 5, dadurch gekennzeichnet, daß die oberen und unteren Stützelemente konisch erweitert sind.

7. Gerät nach einem der Ansprüche 1 bis 6, dadurch gekennzeichnet, daß das starre Zentralstützelement eine Stützstange oder ein Stützstab ist.

8. Gerät nach einem der Ansprüche 1 bis 7, dadurch gekennzeichnet, daß die drehbar angelenkten oberen und unteren Stützelemente eingestellte und verstellbare Sperrmittel zur Begrenzung der Knie- und Beinbewegung aufweisen.

## Revendications

1. Un dispositif orthopédique employé pour la stabilisation du genou dans les armatures moulées pour fractures, comprenant un organe central rigide de support comportant une partie terminale supérieure et une partie terminale inférieure, un organe de support supérieur, partiellement noyé dans le matériau de moulage et articulé axialement de façon pivotante et non glissante à ladite partie terminale supérieure dudit organe central de support, et un organe de support inférieur, partiellement noyé dans le matériau de moulage et articulé axialement de façon pivotante et non glissante à ladite partie terminale inférieur dudit organe central de support, les parties terminales supérieures et inférieures étant adjacentes à l'organe de support aux axes d'articulation, caractérisé en ce que ledit organe central rigide de support a également une partie centrale, le plan de la partie centale étant décalé latéralement par rapport aux plans des parties terminales dans le but d'éviter de comprimer l'articulation du genou du patient.

2. Un dispositif orthopédique employé pour la stabilisation du genou dans les armatures moulées pour fractures, comprenant un organe central rigide de support comportant une partie terminale supérieure et une partie terminale inférieure, un organe de support supérieur, partiellement noyé dans le matériau de moulage et articulé axialement de façon pivotante et non glissante à sa partie terminale inférieure à ladite partie terminale supérieure dudit organe central de support, et un organe de support inférieur, partiellement noyé dans le matériau de moulage et articulé axialement de façon pivotante et non glissante à sa partie terminale supérieure à ladite partie terminale inférieure dudit organe central de support, le plan dudit organe central rigide de support étant adjacent aux parties terminales desdits organes de support supérieur et inférieur, caractérisé en ce que les parties terminales des organes de support supérieur et inférieur sont chacun décalés latéralement par rapport aux plans généraux respectifs des organes de support supérieur et inférieur dans le but d'éviter de comprimer l'articulation du genou du patient.

3. Un dispositif selon la revendication 1 ou 2 dans lequel lesdits organes de support supérieur et inférieur sont noyés dans du matériau de moulage.

4. Un dispositif selon l'une quelconque des revendications 1 à 3 dans lequel il est prévu des ailes de fixation qui sont chacune attachées séparément et individuellement à demeure auxdits organes de support supérieur et inférieur.

5. Un dispositif selon la revendication 4 dans lequel lesdites ailes de fixation sont perpendiculaires auxdits organes de support supérieur et inférieur auxquels elles sont attachées.

6. Un dispositif selon l'une quelconque des revendications 1 à 5 dans lequel lesdits organes de support supérieur et inférieur sont évasés.

7. Un dispositif selon l'une quelconque des revendications 1 à 6 dans lequel ledit organe central rigide de support est une barre ou une tige de support.

8. Un dispositif selon l'une quelconque des revendications 1 à 7 dans lequel lesdits organes de support supérieur et inférieur qui sont articulés axialement de façon pivotante, ont des moyens d'arrêt préréglés et réglables dans le but de limiter le mouvement du genou et de la jambe.

FIG. 1

FIG. 2

FIG. 3

FIG. 4

UPPER

LOWER

FIG. 5

a.

b.

c.

d.

FIG. 6

2